# EUROPEAN PATENT APPLICATION

(11) **EP 3 354 228 A1**
(43) Date of publication of application: **01.08.2018**
(21) Application number: 17153950.5
(22) Date of filing: 31.01.2017
(51) Int. Cl.: A61C 17/22, A46B 15/00

(54) **PACING SYSTEM COMPRISING A TOOTHBRUSH**

(71) Applicant: SCANDERRA GmbH, 8048 Zürich (CH)
(72) Inventor: FLATT, Thomas Eric, 8048 Zürich (CH)
(74) Representative: Pharma Concepts GmbH

(57) **Abstract**

The invention provides a pacing system comprising a signalling device in the form of an electric toothbrush which is adapted to prompt a user, during performance of an oral care operation comprising a first activity related to oral care such as toothbrushing, to also perform a second, not oral care related activity in a manner that is timely coordinated by an output signal emitted by the signalling device. The second activity may for instance be a paced breathing exercise or another health-beneficial exercise.

## Description

### FIELD

The invention relates to a pacing system comprising a signalling device in the form of an electric toothbrush and to a method for prompting and pacing certain health beneficial activities of a user, such as a breathing exercise, during routine oral care activities such as toothbrushing.

### BACKGROUND

Promoted by the fast technical progresses, pace of life has been noted, or perceived, to accelerate profoundly over the last decades; a fact that is to be seen with concern due to the increased level of negative stress and increasing health concerns related to it. The literature offers quite a number of activities which are considered beneficial for the health of users, both in terms of mental health as well physical health and often irrespective of the user's age or gender. For instance, regular physical exercise, even if done over a short time only, is considered one of the main pillars of physical health and wellbeing. Meditation is suggested in order to calm the thoughts and to help users to unwind and gain more focus. Similarly, slow, regular breathing or specifically paced breathing exercises (e.g. yoga breathing exercises) are considered beneficial for relaxation and for increasing the so-called heart-rate variability (HRV), which may reduce the risk of sudden cardiac death. In almost any case, the daily repetition of the exercises, mostly either in the morning or evening or both times is essential to obtain the desired beneficial result.

Numerous mobile phone applications (apps) and/or other dedicated devices entered the market in order to support users in their attempts and efforts to improve time and frequency of their health-beneficial activities. For instance, 'fitness-trackers' in the form of e.g. wristbands or the like remind a user of his/her sport schedule, track their step counts and/or sleep cycles. Apps such as the 'Camera Heart Rate Variability' offer HRV-measurements-and tracking using a smartphone's inbuilt camera. Other apps offer guided meditations or mindfulness exercises along with related reminders which may come in the form acoustical signals or in the form of written text or visuals displayed on the smartphone's screen.

Breath pacing devices are known from the prior art to support the breathing process; providing a sequence of desired respiration cycles corresponding to a natural breathing rhythm and displaying them to a user in form of a perceivable output signal, e.g. a sound changing in intensity or frequency, or a light or visual effect changing in intensity, colour or shape according to the desired respiration cycles. Alternatively, breath pacing devices with a tactile output signal are provided, e.g. an inflatable wristband such as described in EP 2621576 B1 or a portable breath training device with a vibratory disc to be attached to a user's chest such as described in US 2010/0125226 A1. Tactile output signals offer the advantage that the output signal is typically noticeable by the user only and does not disturb other persons in the same room, as would potentially be the case with audible or visual output signals.

However, the drawback of the prior art approaches is that they often require a dedicated device, such as a smartphone, a wristband, a separate breath pacing device. Furthermore, many of the devices or systems fail to remind the user or prompt him/her into action when the device is switched off, if the user first has to actively remember to wear, or carry, the device with or on him/her; or if reminders have not been programmed correctly, e.g. in a smartphone app. Very commonly, compliance with such devices and apps is also low due the lack of time and due to the difficulty to build in new habits into a daily routine. In consequence, users often fail to establish their desired (daily) routines for health-beneficial activities such as exercises related to breathing, meditation, mindfulness and/or sports.

It is therefore an object of the present invention to provide an improved pacing system and method overcoming such drawbacks of the prior art, enabling the user to set-up a (daily) routine for health-beneficial activities more easily than with known pacing systems.

### SUMMARY OF THE INVENTION

According to a first aspect of the present invention, this object is achieved by a pacing system comprising a signalling device in the form of an electric toothbrush for performing oral care operations, the signalling device comprising a controller and at least one signalling member operable by the controller and capable of generating at least one output signal, wherein the signalling device is adapted to prompt the user, during performance of an oral care operation comprising a first activity related to oral care, to perform a second activity in a manner timely coordinated by the at least one output signal, wherein the second activity is different from the first activity and not related to oral care.

The first activity related to oral care may be toothbrushing. The second activity may be selected from a health-beneficial activity such as a paced breathing exercise; a mindfulness and/or meditation exercise; a recital of a prayer and/or mantra; and/or a physical exercise. Optionally, the first and the second activity are performed simultaneously.

The at least one output signal may be selected, for instance, from a tactile signal, an acoustic signal, an optical signal, or any combination thereof. The electric toothbrush may, for instance, have two activity modes, an inactive mode (in which the toothbrush is silent) and an active mode (or brushing mode), and the output signal may be generated by an automatic change between these two activity modes; i.e. a stop-go type signal.

For instance, the output signal may be generated by automatically changing the toothbrush's activity mode according to a sequence comprising (a) a first active mode for a selected time T₁, then (b) an inactive mode for a selected time T₂, followed by (c) a second active mode for a selected time T₃; and optionally (d) a second inactive mode for a selected time T₄ which may serve as the 'end of sequence' indicator, before repeating the sequence. This 'go-stop-go' type sequence may be repeated multiple times during the oral care operation.

In cases where the second activity is a paced breathing exercise, the output signal may be generated by automatically changing the toothbrush's activity mode as follows: (a₁) active brushing for about 2 to 12 seconds, e.g. 4 seconds; (b₁) an inactive mode, or brush stop, for about 1 to 10 seconds, e.g. 4 seconds; (c₁) active brushing again for about 2 to 12 seconds, e.g. 8 seconds; and optionally (d₁) a much briefer second brush stop for about 0.4 to 2 seconds, to indicate the end of the sequence, e.g. 1 second. The breathing exercise for the user may then, for instance, consist of inhaling during the first active brushing phase; holding the breath during the brush stop phase (b₁); and then exhaling during at least the second brush phase (c₁), and optionally during the brief 'end of sequence' brush stop.

Other breathing patterns than this exemplary 4s/4s/8s/1s sequence may, of course, be programmed. The signalling device of the above described pacing system may, for instance, further comprises a user interface (e.g. a button, a small touchscreen, a USB-or Bluetooth-connectivity, or an optical means for reading barcodes or QR-codes), such that the controller of the signalling device can be both pre-programmed to a factory setting and/or programmed by the user via said user interface.

In a second aspect, the present invention provides a method of performing a first activity related to oral care and a second activity different from the first and not related to oral care during an oral care operation in a timely coordinated manner, the method comprising the steps of:
i) providing a pacing system comprising a signalling device in the form of an electric toothbrush, the signalling device comprising a controller and at least one signalling member operable by the controller and capable of generating at least one output signal; and
ii) prompting the user to perform the second activity in a manner timely coordinated by the at least one output signal with the pacing system of step i).

In analogy to the features described for the pacing system described above, the first activity related to oral care may be toothbrushing, and the second activity may be selected from a health-beneficial activity such as a paced breathing exercise; a mindfulness and/or meditation exercise; a recital of a prayer and/or mantra; and/or a physical exercise. According to one embodiment of this method, the output signal is generated by automatically changing the activity mode of the electric toothbrush according to the sequence:
a₁) a first active mode for a selected time T₁ ranging from about 2 seconds to about 12 seconds,
b₁) a first inactive mode for a selected time T₂ ranging from about 1 second to about 10 seconds,
c₁) a second active mode for a selected time T₃ ranging from about 2 seconds to about 12 seconds, and optionally
d₁) a second inactive mode for a selected time T₄ ranging from about 0.4 seconds to about 2 seconds;
and the paced breathing exercise consists of inhaling during T₁, holding the breath during T₂, and exhaling during T₃ and optionally during T₄.

In a third aspect, the present invention provides a kit comprising the pacing system described above, and instructions to the user; e.g. instructions in printed or other readable form, including computer readable forms, such as barcodes, QR-codes or the like.

These and further aspects of the invention will become apparent in more detail from the embodiments described hereinafter.

### BRIEF DESCRIPTION OF THE FIGURE

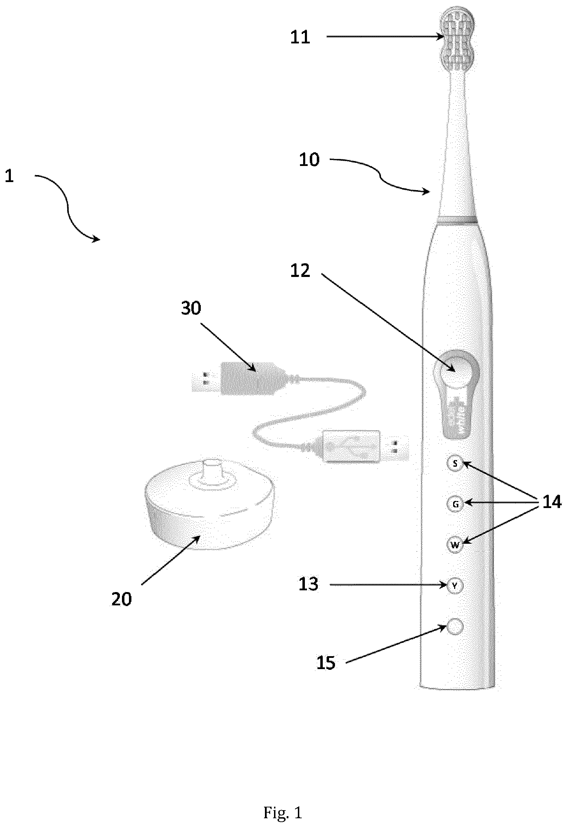

Fig. 1 shows a pacing system (1) according to one embodiment of the present invention, depicting the signalling device in the form of an electric toothbrush (10), a docking/charging station (20), and a USB-cable (30) serving as a user interface.

### DETAILED DESCRIPTION OF THE INVENTION

In a first aspect, the present invention provides a pacing system comprising a signalling device in the form of an electric toothbrush for performing oral care operations, the signalling device comprising a controller and at least one signalling member operable by the controller and capable of generating at least one output signal, wherein the signalling device is adapted to prompt the user, during performance of an oral care operation comprising a first activity related to oral care, to perform a second activity in a manner timely coordinated by the at least one output signal, wherein the second activity is different from the first activity and not related to oral care.

In one embodiment of the invention, the first activity related to oral care is toothbrushing. As used herein the term 'toothbrushing' refers to the actual act and time period in which a user is cleaning, or brushing, his/her teeth with the help of a toothbrush; or in other words the time period in which the user typically holds the toothbrush's brushhead in his/her mouth. The term 'oral care operation' is to be understood in a broader sense and comprises the time period for the actual toothbrushing activity, but also the time periods shortly before or after the act of brushing his/her teeth; e.g. the time when the user applies the toothpaste or when the user rinses the brushhead with water after the toothbrushing.

In a further embodiment the first and the second activity are performed simultaneously; e.g. the toothbrushing (first activity) may be done at the same time as a second, not oral care related activity; for instance, a small physical exercise or a paced breathing exercise. Such exercises are known to provide a health benefit to the user, e.g. by inducing and/or deepening relaxation, advantageously increasing the heart rate variability (HRV), and/or improving muscle tonus; yet they are also often forgotten and not performed regularly enough to benefit from them.

The present invention is based on the idea that most people have been trained since early childhood to routinely brush their teeth daily, preferably two or even three times daily. Thus, providing the signalling device of the inventive pacing system in the form of an electric toothbrush offers the possibility to employ this habitual oral care routine as an easy reminder for a second activity not related to oral care, such as a health-beneficial activity; as well as employing the electric toothbrush as a signalling device and pacing system component which provides at least one output signal to also coordinate this second activity in a timely manner. The signalling device is adapted for this purpose by specific programs operatable by the controller, as will be detailed further below.

In one embodiment, the second activity is selected from a paced breathing exercise; a mindfulness and/or meditation exercise; a recital of a prayer and/or mantra; and/or a physical exercise. Paced breathing exercises, for instances, are common in yogic traditions, prescribing specific sequences of inhalation-, breath-hold- and exhalation manoeuvers with the lengths of each manoeuver varying depending on the purpose. Examples of physical exercises include but are not limited to balancing exercises (e.g. alternatingly standing on one leg at a time, getting up on tiptoes and back onto flat feet again), eye training exercises or muscle relaxation exercises (e.g. flexing and releasing the shoulder muscles).

In that respect, the terms 'in a manner timely coordinated' or 'in a timely coordinated manner' as used herein are supposed to be understood as the second activity being timely controlled both with respect to the first activity, but also being timely controlled within and of its own. For instance, an output signal prompting a user to do the second activity may be timed (i.e. programmed accordingly in the controller) to be emitted a few seconds before the onset of the first activity; e.g. the sound of a meditation gong and/or a recorded instruction (reminding the user of a mindfulness exercise to be done during toothbrushing) could be emitted by the signalling device after the user presses the ON/OFF-button, with the brushhead starting its cleaning movements only after said gong and/or instructions are emitted and the timed number of seconds has passed. Similarly, the second activity may be timely controlled within and of its own if, for instance, a plurality of output signals are emitted in sequence to pace, or guide, the user's breathing (the inhalation- exhalation- and breath-hold periods) throughout the whole time of the toothbrushing activity.

It should be understood that depending on the specifics of the second activity, the signalling device may serve the purpose of a simple reminder in some instances; for instance, the above mentioned meditation gong at the start of an oral care operation to remind the user of a mindfulness practice or a silent recitation of a prayer or mantra while toothbrushing. More typically, though, it serves the purpose of an actual pacing system component which controls the timely coordination of the second activity, thereby improving the safety and efficacy thereof. For instance, the user may be guided through one or more paced breathing sequences in a paced breathing exercise (each sequence, or cycle, including specifically timed periods for inhalations, exhalations and breath-holds); or the user may be guided when and/or how to do a physical exercise while brushing his/her teeth and when to pause from it.

In order to achieve this timely coordination of the second, not oral care related activity, the signalling device is specifically adapted in that its controller operates, or controls, the at least one signalling member in response to at least one protocol, or program, stored in an electronic memory which is connected to the controller, or an integral part thereof. Said programs comprise all required information on e.g. the time periods employed in a specific exercise, such as the lengths of inhalation- and exhalation periods in a paced breathing exercise; as well as the related activity to be performed by the signalling at a specific time Tₓ, such as emitting a sound and which type of sound, emitting a visually detectable light, the wave length of this light, or switching the electric toothbrush on or off, for example.

The programs may either be pre-programmed (factory-setting), and/or they may be programmable by the user via an interface, as will be detailed further below. The signalling member then emits at least one output signal in response to these programs, the output signal being perceived by the user and prompting him/her to perform the second activity, or parts thereof.

The controller may be of any known type, as long as it fits into the housing of an electric toothbrush and is at least capable of reading the electronic memory and controlling the signalling device's signalling member(s), for instance a microcontroller or micro-computer comprising an electronic processor core within an integrated circuit.

In one embodiment, the at least one output signal emitted by the signalling member(s) is selected from a tactile signal, an acoustic signal, an optical signal, or any combination thereof. For example, the signalling device may comprise one or more light-emitting diodes (LEDs), laser diodes, vibration means and/or sound emitting means. In some instances, combinations of output signals may arise inherently; for instance, a vibration signal usually also emits a perceivable acoustic signal at the same time.

In a specific embodiment, the at least one output signal is an acoustic signal. Examples of such acoustic signals include but are not limited to sounds such as the recorded sound of a meditation gong or singing bowl (e.g. in order to remind a user to practice mindfulness during the toothbrushing), beeping sounds of various acoustic pitches and/or intervals (indicative e.g. for specific breathing manoeuvers) or recorded voice instructions to the user when and/or how to perform a specific exercise. It is easily understood that the sound level of such acoustic signals has to be chosen and programmed in such a way that the user is still able to perceive them without problems over the sound level of the active electric toothbrush (i.e. when the brushhead is moving). Optionally, the signalling device may further comprise a volume control to enable the user to choose an appropriate sound level according to his/her own needs.

In another specific embodiment, the at least one output signal is an optical signal, such as light emitted intermittently, light of different wavelengths, and/or light of different intensity. For instance, light of increasing intensity, or brightness, could indicate an inhalation manoeuver, light of decreasing intensity an exhalation manoeuver; and a constant intensity could indicate a breath-hold period. In a further example, light of a first particular wavelength or colour, could indicate an inhalation manoeuver, light of second wavelengths or colour different from the first one could indicate an exhalation manoeuver; and a third colour or no light could indicate a breath-hold period.

In yet another embodiment, the at least one output signal is generated using the at least two activity modes of the electric toothbrush: an inactive mode and an active mode, or brushing mode. The inactive mode refers to the time period in which the toothbrush is silent, i.e. when the brushhead is not moving. The active mode refers to the time period in which the toothbrush is actively brushing, i.e. when the brushhead is moving (e.g. vibrating, oscillating, rotating, pulsating, etc.). In this embodiment, the at least one output signal is generated by an automatic change between these two activity modes. In other words, the output signal is provided in the form of a 'stop-go' type signal, or 'on-off type signal. It should be understood, though, that not necessarily the whole tooth brush is switched off altogether but rather just its brushhead' s movements are put on 'hold' for the programmed time period.

One potential benefit of this tactile output signal approach over acoustic or optical output signals is that these 'stop-go' type signals during the use of the electric toothbrush are very easily perceivable even under circumstances which disturb the perception of acoustic or optical signals, e.g. background noises, very bright surroundings and/or missing mirrors. In fact, such 'stop-go' type signals would also be suited for blind or deaf users, since the presence brushing movements by the brushhead, or lack thereof, is immediately noticeable via the gums, teeth and jawbones.

In a specific embodiment, the at least one output signal is generated by automatically changing the activity mode of the electric toothbrush according to a sequence comprising:
a) a first active mode, or brushing mode, for a selected time T₁,
b) an inactive mode, or brush stop, for a selected time T₂,
c) a second active mode, or brushing mode, for a selected time T₃, and optionally
d) a second inactive mode, or brush stop, for a selected time T₄.

The second inactive mode, or brush stop, of step (d) mainly serves as an 'end of sequence' indicator, e.g. prior to repeating the described sequence. Alternatively, this indication could be provided by another signal, such as a short sound signal or optical signal instead of a second brush stop. For the above described reasons, a tactile signal as an 'end of sequence' indication via the second inactive mode, or brush stop, of step (d) may nonetheless be preferred over acoustic or optical signals.

In one embodiment, the activity mode sequence of the electric toothbrush is repeated multiple times during the oral care operation; such as twice or three or four times or twelve times.

In one of the specific embodiments, the second activity is selected from a paced breathing exercise, and the at least one output signal is generated by automatically changing the activity mode of the electric toothbrush according to the sequence:
a₁) a first active mode, or brushing mode, for a selected time T₁ ranging from about 2 seconds to about 12 seconds, preferably from about 4 seconds to about 10 seconds,
b₁) a first inactive mode, or brush stop, for a selected time T₂ ranging from about 1 second to about 10 seconds, preferably from about 1 second to about 8 seconds,
c₁) a second active mode, or brushing mode, for a selected time T₃ ranging from about 2 seconds to about 12 seconds, preferably from about 6 seconds to about 12 seconds, and optionally
d₁) a second inactive mode, or brush stop, for a selected time T₄ ranging from about 0.4 seconds to about 2 seconds.

In this embodiment, the paced breathing exercise consists of (a₂) inhaling during time T₁; (b₂) holding the breath during time T₂; and (c₂) exhaling during time T₃, and optionally also during time T₄. In other words, the breathing exercise for the user consists of inhaling during the first active brushing phase; holding the breath during the brush stop phase (b₁); and then exhaling during at least the second brush phase (c₁), and optionally during the brief 'end of sequence' indicating brush stop. Preferably, such breathing should be performed through the nose in order to warm up and humidify the air in the nasal airways, filter irritants such as dust, and also in order to avoid unintentional aspiration of saliva and/or toothpaste.

The prior art knows various paced breathing exercises; in particular, the teachings of the yogic tradition comprise numerous breathing exercises with varying lengths of the inhalation-, exhalation- and breath-hold periods, depending for instance on the purpose and/or the targeted organ, all of which can serve as an activity mode pattern of the second activity according to the present invention, in case the total length of the breathing exercise does not significantly exceed the common length of brushing one's teeth.

In exemplary embodiments, the times T₁ for inhalation, T₂ for breath-holding and T₃ for exhalation are selected, respectively, from (T₁ / T₂ / T₃):
- 4 s / 1 s / 4 s;
- 4 s / 2 s / 4 s;
- 4 s / 4 s / 8 s, or
- 4 s / 8 s / 8 s.

If a second brush stop of time period T₄ is employed as an end of sequence indication to the user, the respective exemplary embodiments of the invention are selected from (T₁ / T₂ / T₃ / T₄):
- 4 s / 1 s / 4 s / 0.5 s,
- 4 s / 2 s / 4 s / 0.5 s,
- 4 s / 4 s / 8 s / 0.5 s,
- 4 s / 8 s / 8 s / 0.5 s,
- 4 s / 1 s / 4 s / 1 s,
- 4 s / 2 s / 4 s / 1 s,
- 4 s / 4 s / 8 s /1 s, or
- 4 s / 8 s / 8 s / 1 s.

In one of the preferred embodiments, the times T₁ for inhalation, T₂ for breath-hold and T₃ for exhalation and the second brush stop period T₄ are selected, respectively, from 4 s / 4 s / 8 s / 1 s or 4 s / 4 s / 8 s / 0.5 s. In these embodiments, each sequence is about 17 or 16.5 seconds long, which is about the time suggested for cleaning, or brushing, one of the three surfaces of the teeth per one of the four quadrants; for instance, 17 seconds for the outside surfaces of the teeth in the upper left quadrant, 17 seconds for the inside surfaces and 17 seconds for the masticatory surfaces, or chewing surfaces; then repeating this sequence in the bottom left, upper right and bottom right quadrant. In other words, if the length of the breath pacing sequence has an appropriate length of about 10 to 20 seconds, it may also be employed to pace the toothbrushing itself; one sequence per one of the three surfaces of the teeth in one of the four quadrants; e.g. twelve times 17 seconds in total, i.e. 204 seconds or 3.4 minutes.

In one embodiment, the signalling device according to the present invention further comprises a user interface. This interface may be provided in various forms such as a button, a small touchscreen, an optical means for reading barcodes or QR-codes, a USB-connectivity, a Bluetooth connectivity or a different wireless communication means. Typically, the signalling device will be provided with at least one factory setting (e.g. the "yoga breathing mode", or modes, which may for instance be selectable by the user by pressing the "Y"-button at the electric toothbrush depicted in Fig. 1 either once or repeatedly). In other words, the controller of the signalling device is pre-programmed to this at least one factory setting. In an alternative embodiment, or in addition to the pre-programmed factory-setting, the controller of the signalling device is programmable by the user via the user interface. The benefit of this is that the user is enabled to adapt the reminder- and pacing functions of the signalling device in various aspects; for instance, adding further breathing exercises, adjusting the lengths of the time periods for inhalation, exhalation and/or breath-hold, choosing a preferred sound from a larger selection of acoustic output signals (e.g. a gong, a singing bowl), choosing a specific language for recorded acoustic instructions, and the like.

In a specific embodiment, the signalling device comprises at least one user interface in the form of a USB-connectivity, i.e. a USB-cable is provided as part of the pacing system to allow for connection, communication, and power supply between the signalling device (toothbrush) and computers, e.g. personal computers, laptop devices and mobile devices such as smartphones (see e.g. USB-cable (30) in Fig. 1). Dedicated programs or mobile phone apps may be provided on the computers to allow for easy user control.

In a further specific embodiment, the signalling device comprises at least one user interface in the form an optical means for reading barcodes or QR-codes. The respective barcodes or QR-codes encoding various programs, and thus pacing functions of the signalling device, may be provided on the packaging of the pacing system, on a leaflet inserted in this packaging and/or on the company website of the pacing system provider.

In a second aspect, the present invention provides a method of performing a first activity related to oral care and a second activity different from the first and not related to oral care during an oral care operation in a timely coordinated manner, wherein the method comprises the steps of:
i) providing a pacing system comprising a signalling device in the form of an electric toothbrush, the signalling device comprising a controller and at least one signalling member operable by the controller and capable of generating at least one output signal; and
ii) prompting the user to perform the second activity in a manner timely coordinated by the at least one output signal with the pacing system of step i.

It should be understood that any of the features described for the first aspect of the invention (the pacing system), are equally applicable to the second aspect of the invention (the above described method) and may thus be combined in any technically useful manner. For instance, in one embodiment of the invention, the first activity related to oral care is toothbrushing. In one embodiment, the second activity is selected from a paced breathing exercise; a mindfulness and/or meditation exercise; a recital of a prayer and/or mantra; and/or a physical exercise. In a further embodiment, the first and the second activity are performed simultaneously.

In a specific embodiment of the method of the present invention, the first activity related to oral care is toothbrushing, and the second activity is selected from a paced breathing exercise.

In one embodiment of the method, the at least one output signal employed for prompting a user to perform the second activity in a timely controlled manner is selected from a tactile signal, an acoustic signal, an optical signal, or any combination thereof.

In one of the preferred embodiments of the method, the at least one output signal is a tactile signal such as it may be generated by automatically changing the activity mode of the electric toothbrush according to the sequence:
a) a first active mode, or brushing mode, for a selected time T₁,
b) an inactive mode, or brush stop, for a selected time T₂,
c) a second active mode, or brushing mode, for a selected time T₃, and optionally
d) a second inactive mode, or brush stop, for a selected time T₄.

Said activity mode sequence of the electric toothbrush may be repeated multiple times during the oral care operation

In a further specific embodiment of the method, the at least one output signal is generated by automatically changing the activity mode of the electric toothbrush according to the sequence:
a₁) a first active mode for a selected time T₁ ranging from about 2 seconds to about 12 seconds,
b₁) a first inactive mode for a selected time T₂ ranging from about 1 second to about 10 seconds,
c₁) a second active mode for a selected time T₃ ranging from about 2 seconds to about 12 seconds, and optionally
d₁) a second inactive mode for a selected time T₄ ranging from about 0.4 seconds to about 2 seconds; and
the paced breathing exercise consists of inhaling during T₁, holding the breath during T₂, and exhaling during T₃ and optionally during T₄.

In a third aspect, the present invention provides a kit comprising the pacing system described above, and instructions to the user, e.g. instructions in printed or other readable form, including computer readable forms, such as barcodes, QR-codes or the like. In one specific embodiment, written instructions are printed on the packaging in which the pacing system and/or provided as a leaflet within said packaging. The instructions may for instance be provided to introduce a user to the general concept of using the 'stop-go'-modes of the electric toothbrush for pacing his/her breathing manoeuvers.

It should again be understood that any of the features described for the first aspect of the invention (the pacing system), are equally applicable to the third aspect of the invention (the kit) and may thus be combined in any technically useful manner.

### DETAILED DESCRIPTION OF THE FIGURE

Fig. 1 shows a pacing system (1) according to one embodiment of the present invention, depicting the signalling device in the form of an electric toothbrush (10), the toothbrush having a brushhead (11), an ON/OFF-button (12), a "Y-button" (13; "Y" referring to "yoga", or yogic breathing exercises), three brush-mode buttons (14), as well as an ON/OFF- and battery indicator (15; e.g. in the form of an LED). Also shown are a docking/ charging station (20) for the electric toothbrush (10), and a USB-cable (30) serving as a user interface for the electric toothbrush (10).

In this depicted embodiment, the user has the option to select via separate buttons whether he/she wants to perform the programmed at least one breathing exercise, by pressing (or not pressing) the "Y-button" (13) once the toothbrush (10) is switched on via the ON/OFF-button (12). In addition, the user may further select a specific brush-mode by pressing one of the three brush-mode buttons (14): S refers to a "soft" / "sensitive" brush mode, G to a "general" brush mode and W to a "whitening" brush mode, the three modes varying in the intensity and speed of the brushhead' s movements.

While the invention has been illustrated and described in detail in the figure and the foregoing description, the figure and description are to be considered illustrative or exemplary and not restrictive; the invention is not limited to the disclosed embodiments. Other variations to the disclosed embodiments can be understood and effected by those skilled in the art in practicing the claimed invention, from a study of the description, the figures, and the appended claims. As used herein, the word 'comprising' does not exclude other elements or steps, and the indefinite article 'a' or 'an' does not exclude a plurality. The mere fact that certain measures are recited in mutually different dependent claims does not indicate that a combination of these measures cannot be used to advantage. Any reference signs in the claims should not be construed as limiting the scope.

## Claims

1. A pacing system comprising a signalling device in the form of an electric toothbrush for performing oral care operations, the signalling device comprising a controller and at least one signalling member operable by the controller and capable of generating at least one output signal,
wherein the signalling device is adapted to prompt the user, during performance of an oral care operation comprising a first activity related to oral care, to perform a second activity in a manner timely coordinated by the at least one output signal,
wherein the second activity is different from the first activity and not related to oral care.

2. The pacing system according to claim 1, wherein the first activity related to oral care is toothbrushing.

3. The pacing system according to claim 1 or 2, wherein the first and the second activity are performed simultaneously.

4. The pacing system according to any one of claims 1 to 3, wherein the at least one output signal is selected from a tactile signal, an acoustic signal, an optical signal, or any combination thereof.

5. The pacing system according to any one of claims 1 to 4, wherein the electric toothbrush has at least two activity modes, an inactive mode and an active mode, and wherein the at least one output signal is generated by an automatic change between these two activity modes.

6. The pacing system according to any one of claims 1 to 5, wherein the at least one output signal is generated by automatically changing the activity mode of the electric toothbrush according to a sequence comprising:
a) a first active mode for a selected time T₁,
b) an inactive mode for a selected time T₂,
c) a second active mode for a selected time T₃, and optionally
d) a second inactive mode for a selected time T₄.

7. The pacing system according to claim 6, wherein the activity mode sequence of the electric toothbrush is repeated multiple times during the oral care operation.

8. The pacing system according to any one of claims 1 to 7, wherein the second activity is selected from a paced breathing exercise; a mindfulness and/or meditation exercise; a recital of a prayer and/or mantra; and/or a physical exercise.

9. The pacing system according to any one of claims 1 to 8, wherein the second activity is selected from a paced breathing exercise, and wherein the at least one output signal is generated by automatically changing the activity mode of the electric toothbrush according to the sequence:
a₁) a first active mode for a selected time T₁ ranging from about 2 seconds to about 12 seconds,
b₁) a first inactive mode for a selected time T₂ ranging from about 1 second to about 10 seconds,
c₁) a second active mode for a selected time T₃ ranging from about 2 seconds to about 12 seconds, and optionally
d₁) a second inactive mode for a selected time T₄ ranging from about 0.4 seconds to about 2 seconds.

10. The pacing system according to claim 9, wherein the paced breathing exercise consists of:
a₂) inhaling during T₁;
b₂) holding the breath during T₂; and
c₂) exhaling during T₃ and optionally during T₄.

11. The pacing system according to any one of claims 1 to 10, wherein the signalling device further comprises a user interface; and
wherein the controller of the signalling device is pre-programmed to a factory setting, and/or programmable by the user via the user interface.

12. A method of performing a first activity related to oral care and a second activity different from the first and not related to oral care during an oral care operation in a timely coordinated manner, wherein the method comprises the steps of:
i) providing a pacing system comprising a signalling device in the form of an electric toothbrush, the signalling device comprising a controller and at least one signalling member operable by the controller and capable of generating at least one output signal; and
ii) prompting the user to perform the second activity in a manner timely coordinated by the at least one output signal with the pacing system of step i).

13. The method of claim 12, wherein the first activity related to oral care is toothbrushing, and wherein the second activity is selected from a paced breathing exercise.

14. The method of claim 13, wherein the at least one output signal is generated by automatically changing the activity mode of the electric toothbrush according to the sequence:
a₁) a first active mode for a selected time T₁ ranging from about 2 seconds to about 12 seconds,
b₁) a first inactive mode for a selected time T₂ ranging from about 1 second to about 10 seconds,
c₁) a second active mode for a selected time T₃ ranging from about 2 seconds to about 12 seconds, and optionally
d₁) a second inactive mode for a selected time T₄ ranging from about 0.4 seconds to about 2 seconds; and
wherein the paced breathing exercise consists of inhaling during T₁, holding the breath during T₂, and exhaling during T₃ and optionally T₄.

15. A kit comprising the pacing system according to any one of claims 1 to 11, and instructions to the user.
